(19) Europäisches Patentamt
European Patent Office
Office européen des brevets

(11) EP 4 227 451 B1

# (12) EUROPEAN PATENT SPECIFICATION

(45) Date of publication and mention of the grant of the patent:
**25.02.2026 Bulletin 2026/09**

(21) Application number: **21877630.0**

(22) Date of filing: **05.10.2021**

(51) International Patent Classification (IPC):
***D01F 6/46*** *(2006.01)*
***D04H 3/007*** *(2012.01)*
***D04H 3/16*** *(2006.01)*
***A61F 13/514*** *(2006.01)*
***A61L 15/24*** *(2006.01)*
***A61L 15/52*** *(2006.01)*
***A61L 15/34*** *(2006.01)*
***B32B 5/02*** *(2006.01)*
***B32B 5/26*** *(2006.01)*
***D01F 1/10*** *(2006.01)*
***D04H 1/4291*** *(2012.01)*
***D04H 1/56*** *(2006.01)*

(52) Cooperative Patent Classification (CPC):
(C-Sets available)
**D01F 6/46; A61F 13/51401; A61L 15/24; A61L 15/34; A61L 15/52; B32B 5/022; B32B 5/269; D04H 1/4291; D04H 1/56; D04H 3/007; D04H 3/16;**
A61F 2013/51452; B32B 2250/20; B32B 2262/0253; B32B 2307/718; (Cont.)

(86) International application number:
**PCT/JP2021/036872**

(87) International publication number:
**WO 2022/075331 (14.04.2022 Gazette 2022/15)**

## (54) NONWOVEN FABRIC, AND LEAKPROOF SHEET FOR ABSORBENT ARTICLE COMPRISING SAME

VLIESSTOFF UND LECKSICHERE LAGE FÜR SAUGFÄHGEN ARTIKEL DAMIT

TISSU NON TISSÉ ET FEUILLE ÉTANCHE POUR ARTICLE ABSORBANT LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2020 JP 2020169389**
**22.03.2021 JP 2021046894**

(43) Date of publication of application:
**16.08.2023 Bulletin 2023/33**

(73) Proprietor: **Kao Corporation**
**Chuo-ku**
**Tokyo 103-8210 (JP)**

(72) Inventors:
- **MIZUKOSHI, Yutaro**
  **Haga-gun, Tochigi 321-3497 (JP)**
- **MAEDA, Naruaki**
  **Haga-gun, Tochigi 321-3497 (JP)**
- **SASE, Masakazu**
  **Haga-gun, Tochigi 321-3497 (JP)**

(74) Representative: **Vossius & Partner**
**Patentanwälte Rechtsanwälte mbB**
**Siebertstraße 3**
**81675 München (DE)**

(56) References cited:
**EP-A1- 2 266 514**
**EP-A1- 2 901 977**
**JP-A- 2004 277 467**
**JP-A- 2009 227 931**
**JP-A- 2012 530 855**
**JP-A- 2012 531 530**
**JP-A- 2013 159 884**
**JP-A- 2016 089 009**
**JP-A- 2018 503 751**
**JP-A- 2019 509 406**
**JP-B1- S 478 932**
**US-A1- 2012 109 090**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 227 451 B1

(52) Cooperative Patent Classification (CPC): (Cont.)
B32B 2307/7265; D01F 1/10

C-Sets
**A61L 15/24, C08L 23/12**

## Description

### Technical Field

The present invention relates to a nonwoven fabric. The present invention also relates to a leakproof sheet for an absorbent article, the leakproof sheet including the nonwoven fabric.

### Background Art

In absorbent articles such as diapers and napkins and sanitary articles such as wound dressings and medical gowns, a nonwoven fabric is typically used as the barrier material to body fluids such as urine and blood. It is generally known that a nonwoven fabric having a smaller fiber diameter or having a smaller gap among fibers delivers a higher performance as the barrier material. This mechanism is effective for a liquid having a relatively high surface tension but may be insufficient for a liquid having a low surface tension, such as loose feces. To further improve the barrier properties of a nonwoven fabric, a reduction in surface tension of fibers is effective.
As a method of reducing the surface tension, treating the surface of fibers with a silicon compound such as silicone or a fluorine compound has been generally known. Using such a compound, however, is unfavorable in view of heat resistance, storage stability, safety of the human body, and others. In place of these compounds, adding a triglyceride to a resin has been disclosed (see Patent Literatures 1 to 4).

### Citation List

#### Patent Literatures

Patent Literature 1: US2012/100772A1
Patent Literature 2: US2014/272223A1
Patent Literature 3: US2020/129345A1
Patent Literature 4: US2019/070045A1

Further prior art is knwon from EP 2266514, US 2012/109090, EP 2901977, JP 2009 227931, JP 2004 277467, JP 2012 530855, JP 2013 159884, JP2012 531530, JP 2019 509406, JP 2018 503751, JP S 478932 and JP 2016 089009.

### Summary of Invention

The present invention provides a nonwoven fabric including fibers containing: a thermoplastic resin having capability to be formed into fibers; and a triglyceride.
The thermoplastic resin preferably includes a polyolefin resin.
The triglyceride preferably includes

(a) a mixture of a triglyceride at least containing, in one molecule, a group derived from a fatty acid having 16 carbon atoms and a triglyceride at least containing, in one molecule, a group derived from a fatty acid having 18 carbon atoms, or
(b) a triglyceride at least containing, in one molecule, a group derived from a fatty acid having 16 carbon atoms and a group derived from a fatty acid having 18 carbon atoms.

The present invention provides a water repellent composition to be contained in a resin, the water repellent composition containing a triglyceride.
The triglyceride preferably includes

(a) a mixture of a triglyceride at least containing, in one molecule, a group derived from a fatty acid having 16 carbon atoms and a triglyceride at least containing, in one molecule, a group derived from a fatty acid having 18 carbon atoms, or
(b) a triglyceride at least containing, in one molecule, a group derived from a fatty acid having 16 carbon atoms and a group derived from a fatty acid having 18 carbon atoms.

Description of Embodiments

When a nonwoven fabric including fibers containing a triglyceride comes into contact with a large amount of a liquid having a low surface tension or is in contact with a liquid having a low surface tension for a long time, the liquid may seep from the nonwoven fabric unfortunately. The present invention therefore relates to a nonwoven fabric having stronger barrier properties to liquid than conventional nonwoven fabrics.

The present invention will now be described on the basis of preferred embodiments. The present invention relates to a nonwoven fabric. The nonwoven fabric of the present invention widely encompasses nonwoven fabrics produced by various methods. As the production method of a nonwoven fabric, various methods are known, including a melt blown method, a spunbond method, an air-through method, an airlaid method, a spunlace method, a needle punching method, an electrospinning method, a chemical bonding method, and a thermal bonding method, and the nonwoven fabric of the present invention encompasses nonwoven fabrics produced by any of these methods. The production method of the nonwoven fabric is not limited to the above, and a nonwoven fabric produced by any other method and a nonwoven fabric produced by a combination of any of these methods are also included in the present invention. The nonwoven fabric of the present invention has strong barrier properties to liquid as described later, and it is therefore particularly advantageous that the nonwoven fabric of the present invention includes a meltblown nonwoven fabric, which easily develops the strong barrier properties, or a composite nonwoven fabric having a meltblown layer. Examples of the composite nonwoven fabric having a meltblown layer include a meltblown-spunbond composite nonwoven fabric and a spunbond-meltblown-spunbond composite nonwoven fabric.

When the nonwoven fabric of the present invention is a composite nonwoven fabric at least containing a spunbond layer and a meltblown layer, the mass ratio of the spunbond layer to the whole of the nonwoven fabric is preferably 50% by mass or more and 95% by mass or less in view of improving the barrier properties to liquid. In view of more markedly enhancing this advantage, the mass ratio of the spunbond layer to the whole of the nonwoven fabric is more preferably 50% by mass or more and 90% by mass or less and even more preferably 50% by mass or more and 85% by mass or less.

The mass ratio of the meltblown layer to the whole of the nonwoven fabric is preferably 5% by mass or more and 50% by mass or less in view of improving the barrier properties to liquid. In view of more markedly enhancing the advantage, the mass ratio of the meltblown layer to the whole of the nonwoven fabric is more preferably 10% by mass or more and 50% by mass or less and even more preferably 15% by mass or more and 50% by mass or less.

One of the characteristics of the nonwoven fabric of the present invention is the constituent fibers thereof. Specifically, the fibers included in the nonwoven fabric of the present invention contain a thermoplastic resin having capability to be formed into fibers and a triglyceride. As the thermoplastic resin having capability to be formed into fibers, various resins are known in the art, and in the present invention, a polyolefin resin is preferably used as the thermoplastic resin in view of producing a nonwoven fabric having strong barrier properties to liquid.

As the polyolefin resin, a homopolymer or a copolymer of a lower olefin such as ethylene and propylene may be used, for example. Specific examples include polyethylene, polypropylene, and ethylene-α-olefin copolymer.

Examples of the polyethylene include high-density polyethylene, low-density polyethylene, and linear low-density polyethylene.

Examples of the polypropylene include isotactic polypropylene and syndiotactic polypropylene. As the polypropylene, low stereoregularity homopolypropylene may also be used. The low stereoregularity homopolypropylene is a homopolypropylene having a mesopentad fraction (mmmm [%]) of 90% or less, which is an index of stereoregularity and is determined by $^{13}C$-NMR.

Examples of the ethylene-α-olefin copolymer include a copolymer of ethylene with an α-olefin such as propylene, 1-butene, 1-pentene, and 1-hexene. Examples of the form of the copolymer include a random copolymer, a block copolymer, and a graft copolymer. In particular, a random copolymer of ethylene and propylene (hereinafter also referred to as "random propylene copolymer") is preferably used.

The proportion of the ethylene in the random propylene copolymer is preferably 2% by mass or more and 70% by mass or less, more preferably 4% by mass or more and 60% by mass or less, and even more preferably 6% by mass or more and 50% by mass or less, in view of improving the barrier properties of the nonwoven fabric to liquid.

Of these polyolefin resins, a polypropylene and a polyolefin containing propylene as a copolymerized component are preferably used in view of good capability to be formed into fiber and strong barrier properties of a nonwoven fabric to be produced to liquid.

The polyolefin resin is also preferably a blend of a random propylene copolymer or a low stereoregularity homopolypropylene with a homopolypropylene other than the low stereoregularity homopolypropylene (hereinafter, a homopolypropylene other than the low stereoregularity homopolypropylene is simply referred to as a "homopolypropylene" unless otherwise specified). In particular, in view of strong barrier properties of the nonwoven fabric to liquid, the polyolefin resin is preferably a blend of a random propylene copolymer and a homopolypropylene.

The proportion of the random propylene copolymer or the low stereoregularity homopolypropylene in the fibers

included in the nonwoven fabric of the present invention (in the fibers included in a meltblown nonwoven fabric when the nonwoven fabric of the present invention has the meltblown nonwoven fabric as described later) is preferably 5% by mass or more in view of further improving the barrier properties of the nonwoven fabric to liquid, and is more preferably 7% by mass or more, even more preferably 9% by mass or more, and further preferably 15% by mass or more, in view of furthermore improving the barrier properties.

The proportion is preferably less than 60% by mass in view of improving the fiber spinning performance, and is more preferably 45% by mass or less, and even more preferably 30% by mass or less, in view of further improving the spinning performance.

The fibers included in the nonwoven fabric of the present invention may contain only a single type of the polyolefin resin or may contain two or more types of the polyolefin resins.

The fibers included in the nonwoven fabric of the present invention may contain, as the component resin thereof, only the above-described polyolefin resin or may contain another thermoplastic resin in addition to the polyolefin resin. In the latter case, the fibers preferably contain 50% by mass or more of the above-described polyolefin resin, as the component resin.

The triglyceride, which is another component contained in the fibers included in the nonwoven fabric of the present invention, is used to reduce the surface tension of the fibers included in the nonwoven fabric of the present invention to thereby improve the barrier properties of the nonwoven fabric to liquid. The triglyceride may adhere to the surfaces of the fibers, or may be kneaded with the resin for the fibers so that the triglyceride is present in the resin.

As the triglyceride, a triglyceride having groups derived from a saturated fatty acid, a triglyceride having groups derived from an unsaturated fatty acid, or a triglyceride having groups derived from a saturated fatty acid and an unsaturated fatty acid may be used, for example. In each case, the fatty acid may have 12 or more and 24 or less carbon atoms, for example. In view of further improving the barrier properties of the nonwoven fabric to liquid, the fatty acid preferably has 14 or more and 22 or less carbon atoms and more preferably 16 or more and 20 or less carbon atoms.

A single kind of triglyceride may be used, or a mixture of a plurality types of triglycerides may be used.

The fibers included in the nonwoven fabric of the present invention may contain only the triglyceride as the glyceride or may contain a monoglyceride and/or a diglyceride in addition to the triglyceride as long as the intended effect of the invention is exhibited. In view of improving the barrier properties of the nonwoven fabric of the present invention to liquid, only the triglyceride is preferably used as the glyceride.

The triglyceride used in the present invention is characterized by having a fatty acid residue having the particular number of carbons. Using such a triglyceride can improve the barrier properties of the nonwoven fabric to liquid. Specifically, the following (a) or (b) is preferably used as the triglyceride.

(a) Triglyceride including a mixture of a triglyceride at least containing, in one molecule, a group derived from a fatty acid having 16 carbon atoms and a triglyceride at least containing, in one molecule, a group derived from a fatty acid having 18 carbon atoms.

(b) Triglyceride including a triglyceride at least containing, in one molecule, a group derived from a fatty acid having 16 carbon atoms and a group derived from a fatty acid having 18 carbon atoms.

For the case (a), two or more of triglycerides are used. Of the plurality of triglycerides, at least one triglyceride contains, in one molecule, at least one group derived from a fatty acid (i.e., a saturated fatty acid or an unsaturated fatty acid) having 16 carbon atoms (the triglyceride is also referred to as "triglyceride 16"). The triglyceride 16 preferably contains, in one molecule, at least one group derived from a fatty acid having 16 carbon atoms. The triglyceride 16 may contain, in one molecule, a single group derived from a fatty acid having 16 carbon atoms (the triglyceride is also referred to as "triglyceride P"), two such groups (the triglyceride is also referred to as "triglyceride PP"), or three such groups (the triglyceride is also referred to as "triglyceride PPP").

The other fatty acid residue(s) in the triglyceride P and the triglyceride PP may be of any type, and may be, for example, a saturated fatty acid or unsaturated fatty acid residue having 12 or more and 24 or less carbon atoms.

The triglyceride 16 may consists of the triglyceride P, may consists of the triglyceride PP, or may consists of the triglyceride PPP.

The triglyceride 16 may be a combination of two or more of the triglyceride P, the triglyceride PP, and the triglyceride PPP. For example, the triglyceride 16 may be a combination of the triglyceride P and the triglyceride PP, a combination of the triglyceride P and the triglyceride PPP, a combination of the triglyceride PP and the triglyceride PPP, or a combination of the triglyceride P, the triglyceride PP, and the triglyceride PPP.

Of the plurality of triglycerides in the case (a), at least one triglyceride contains, in one molecule, at least one group derived from a fatty acid (i.e., a saturated fatty acid or an unsaturated fatty acid) having 18 carbon atoms (the triglyceride is also referred to as "triglyceride 18"). The triglyceride 18 preferably contains, in one molecule, at least one group derived from a fatty acid having 18 carbon atoms. The triglyceride 18 may contain, in one molecule, a single group derived from a fatty acid having 18 carbon atoms (the triglyceride is also referred to as "triglyceride S"), two such groups

(the triglyceride is also referred to as "triglyceride SS"), or three such groups (the triglyceride is also referred to as "triglyceride SSS").

The other fatty acid residue(s) in the triglyceride S and the triglyceride SS may be of any type, and may be, for example, a saturated fatty acid or unsaturated fatty acid residue having 12 or more and 24 or less carbon atoms.

The triglyceride 18 may consists of the triglyceride S, may consists of the triglyceride SS, or may consists of the triglyceride SSS.

The triglyceride 18 may be a combination of two or more of the triglyceride S, the triglyceride SS, and the triglyceride SSS. For example, the triglyceride 18 may be a combination of the triglyceride S and the triglyceride SS, a combination of the triglyceride S and the triglyceride SSS, a combination of the triglyceride SS and the triglyceride SSS, or a combination of the triglyceride S, the triglyceride SS, and the triglyceride SSS.

In the case (a), the triglyceride may consist of the triglyceride 16 and the triglyceride 18, or may include the triglyceride 16, the triglyceride 18, and an additional triglyceride. Examples of the additional triglyceride include triglycerides having neither a group derived from a fatty acid having 16 carbon atoms nor a group derived from a fatty acid having 18 carbon atoms.

The group derived from a fatty acid having 16 carbon atoms is designated as "P", the group derived from a fatty acid having 18 carbon atoms is designated as "S", and groups derived from other fatty acids are designated as "X" and "Y". The combination of groups derived from fatty acids included in the triglyceride is any of PPX (i.e., triglyceride PP), SSX (i.e., triglyceride SS), PXY (i.e., triglyceride P), and SXY (i.e., triglyceride S). The triglycerides represented by PPX, SSX, PXY, and SXY have the following structures (A) to (J).

[Chemical Formula 1]

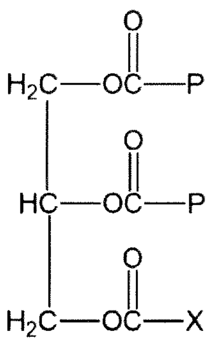
(A)

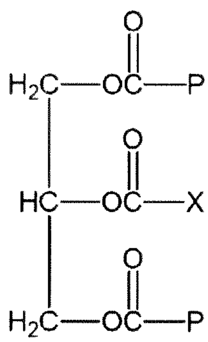
(B)

[Chemical Formula 2]

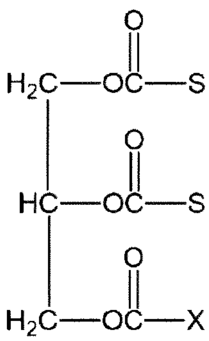
(C)

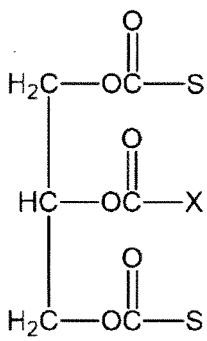
(D)

[Chemical Formula 3]

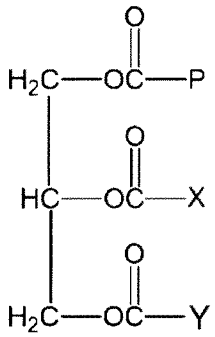 (E) 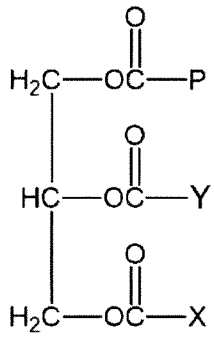 (F) 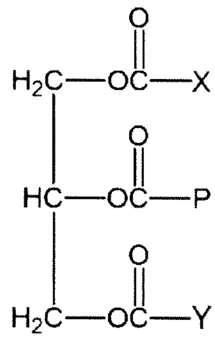 (G)

[Chemical Formula 4]

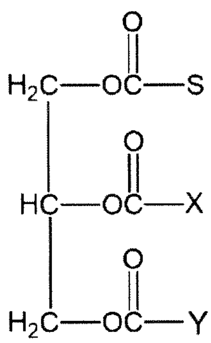 (H) 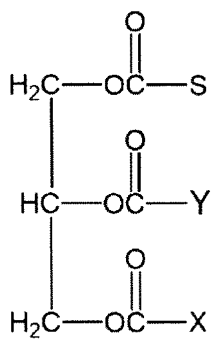 (I) 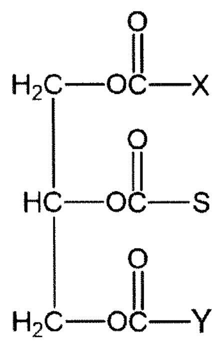 (J)

In view of producing a nonwoven fabric having stronger barrier properties to liquid, the triglyceride to be used preferably consists of the triglyceride 16 and the triglyceride 18.

For the case (b), at least one type of triglyceride may be used. The triglyceride contains, in one molecule, at least one group derived from a fatty acid (i.e., a saturated fatty acid or an unsaturated fatty acid) having 16 carbon atoms and at least one group derived from a fatty acid (i.e., a saturated fatty acid or an unsaturated fatty acid) having 18 carbon atoms. In particular, the triglyceride preferably contains, in one molecule, at least one group derived from a saturated fatty acid having 16 carbon atoms and at least one group derived from a saturated fatty acid having 18 carbon atoms in view of producing a nonwoven fabric having stronger barrier properties to liquid.

The group derived from a fatty acid having 16 carbon atoms is designated as "P", the group derived from a fatty acid having 18 carbon atoms is designated as "S", and a group derived from another fatty acid is designated as "X". In the case (b), the combination of aliphatic groups included in the triglyceride is SSP, SPP, or SPX. The triglyceride represented by SPX has any of the following structures (K) to (M). The same is also applied to SSP and SPP.

[Chemical Formula 5]

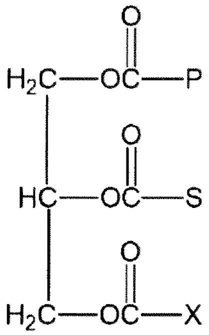 (K) 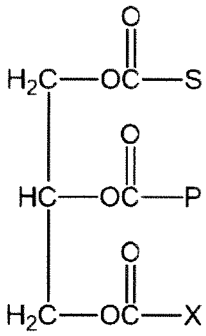 (L) 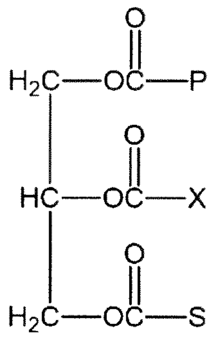 (M)

In the case (b), the triglyceride may consist of a triglyceride containing, in one molecule, at least one group derived from a fatty acid having 16 carbon atoms and at least one group derived from a fatty acid having 18 carbon atoms. Alternatively, the triglyceride may include: a triglyceride containing at least one group derived from a fatty acid having 16 carbon atoms and containing at least one group derived from a fatty acid having 18 carbon atoms; and an additional glyceride. Examples of the additional triglyceride include triglycerides having, in one molecule, neither a group derived from a fatty acid having 16 carbon atoms nor a group derived from a fatty acid having 18 carbon atoms. In view of producing a nonwoven fabric having stronger barrier properties to liquid, the triglyceride to be used preferably consists of a triglyceride containing, in one molecule, at least one group derived from a fatty acid having 16 carbon atoms and at least one group derived from a fatty acid having 18 carbon atoms.

In the present invention, a combination of (a) and (b) may be used. Specifically, a combination of triglyceride PPP, triglyceride SSS, and SPX may be used. Alternatively, a combination of triglyceride PPP, triglyceride SSS, and SSP or a combination of triglyceride PPP, triglyceride SSS, and SPP may be used. Alternatively, a combination of triglyceride PPX, triglyceride SSX, and triglyceride SPX, a combination of triglyceride PPX, triglyceride SSX, and triglyceride SSP, a combination of triglyceride PPX, triglyceride SSX, and triglyceride SPP, a combination of triglyceride PXY, triglyceride SXY, and triglyceride SPX, a combination of triglyceride PXY, triglyceride SXY, and triglyceride SSP, or a combination of triglyceride PXY, triglyceride SXY, and triglyceride SPP may be used.

In each of the cases (a) and (b), the triglyceride used in the present invention preferably contains no group derived from an unsaturated fatty acid in view of improving the barrier properties of the nonwoven fabric of the present invention to liquid. Herein, containing no group derived from an unsaturated fatty acid encompasses both the case in which absolutely no group derived from an unsaturated fatty acid is contained and the case in which a small amount of an unsaturated fatty acid is inevitably contained. The case in which a small amount of an unsaturated fatty acid is inevitably contained may be, for example, a case in which the proportion of the group derived from an unsaturated fatty acid is 2% by mass or less in the total amount of groups derived from fatty acids contained in all the triglycerides contained in fibers.

For all triglycerides contained in the constituent fibers of the nonwoven fabric of the present invention, it is preferable to adjust the proportion of the group derived from a fatty acid having 16 carbon atoms and the proportion of the group derived from a fatty acid having 18 carbon atoms, in view of further improving the barrier properties of the nonwoven fabric to liquid.

For all triglycerides contained in the constituent fibers of the nonwoven fabric, the proportion of the group derived from a fatty acid having 16 carbon atoms in the total amount of groups derived from fatty acids is preferably 1% by mass or more in view of further improving the barrier properties of the nonwoven fabric to liquid, and is more preferably 10% by mass or more, even more preferably 15% by mass or more, further preferably 20% by mass or more, and furthermore preferably 25% by mass or more in view of furthermore improving the barrier properties.

The proportion is preferably less than 55% by mass in view of further improving the barrier properties of the nonwoven fabric to liquid, and is more preferably 50% by mass or less and even more preferably 45% by mass or less in view of furthermore improving the barrier properties.

The proportion is preferably not less than 1% by mass and less than 55% by mass, more preferably not less than 10% by mass and less than 55% by mass, even more preferably not less than 15% by mass and less than 55% by mass, further preferably 20% by mass or more and 50% by mass or less, and particularly preferably 25% by mass or more and 45% by mass or less.

For all triglycerides contained in the constituent fibers of the nonwoven fabric, the proportion of the group derived from a fatty acid having 18 carbon atoms in the total amount of groups derived from fatty acids is preferably 5% by mass or more in view of further improving the barrier properties of the nonwoven fabric to liquid, and is more preferably 35% by mass or more, and even more preferably 45% by mass or more in view of furthermore improving the barrier properties. The proportion is further preferably 50% by mass or more and furthermore preferably 55% by mass or more.

The proportion is preferably less than 90% by mass in view of further improving the barrier properties of the nonwoven fabric to liquid, and is more preferably 70% by mass or less and even more preferably 65% by mass or less in

view of furthermore improving the barrier properties.

The proportion is preferably not less than 5% by mass and less than 90% by mass, more preferably not less than 35% by mass and less than 90% by mass, even more preferably not less than 45% by mass and less than 90% by mass, further preferably 50% by mass or more and 70% by mass or less, and furthermore preferably 55% by mass or more and 65% by mass or less.

As for the ratio between the group derived from a fatty acid having 16 carbon atoms to the group derived from a fatty acid having 18 carbon atoms, the mass ratio of the group derived from a fatty acid having 18 carbon atoms to the group derived from a fatty acid having 16 carbon atoms (C18/C16) in all triglycerides contained in the fibers is preferably 1.0 or more and 15.0 or less in view of further improving the barrier properties of the nonwoven fabric to liquid. In view of more markedly enhancing this advantage, the mass ratio of the group derived from a fatty acid having 18 carbon atoms to the group derived from a fatty acid having 16 carbon atoms (C18/C16) is more preferably 1.2 or more and 10.0 or less, even more preferably 1.3 or more and 5.0 or less, and further preferably 1.3 or more and 2.7 or less.

The proportion of the group derived from a fatty acid having 16 carbon atoms and the proportion of the group derived from a fatty acid having 18 carbon atoms in the total amount of the groups derived from fatty acids in all triglycerides contained in the fibers are determined by the following method.

A good solvent for triglycerides, such as toluene, is used to extract triglycerides on the surface of the fibers included in a nonwoven fabric.

The ester bonds of the extracted triglycerides are hydrolyzed with an alkali, and methyl esters of the fatty acids are quantified by gas chromatography.

Whether a triglyceride has, in one molecule, alkyl chains having the different numbers of carbon atoms can be determined by TOF-MS (time-of-flight mass spectrometry). Specifically, the molecular weight distribution of a triglyceride is measured by TOF-MS, and whether a molecule contains alkyl groups having the different numbers of carbon atoms is determined from the molecular weight of the molecule. Whether compounds having the same molecular weight have, in one molecule, alkyl chains having the different numbers of carbon atoms can be determined using a tandem mass spectrometer (MS/MS) as the mass spectrometry. Specifically, a particular ion is selected in the first mass separation unit. and fragment ions formed by collision of the particular ion with an inert gas were separated and detected in the second mass separation unit.

In the nonwoven fabric of the present invention, triglyceride (a) and/or triglyceride (b) are preferably contained in an amount of 1% by mass or more based the fibers containing triglycerides, in view of further improving the barrier properties of the nonwoven fabric to liquid, and are more preferably contained in an amount of 5% by mass or more and even more preferably 9% by mass or more in view of furthermore improving the barrier properties.

The proportion of triglyceride(s) in the fibers containing the triglyceride(s) is preferably 30% by mass or less in view of good spinning performance and of good texture of the nonwoven fabric, and is more preferably 20% by mass or less, even more preferably 15% by mass or less, and further preferably 14% by mass or less.

The proportion of triglyceride(s) in the fibers containing the triglyceride(s) is preferably not less than 1% by mass and less than 30% by mass, more preferably 5% by mass or more and 20% by mass or less, even more preferably 9% by mass or more and 15% by mass or less, and further preferably 9% by mass or more and 14% by mass or less.

An exemplary method of incorporating a triglyceride in fibers included in the nonwoven fabric includes melting and kneading the thermoplastic resin for the fibers with the triglyceride to blend the triglyceride into the thermoplastic resin. In other words, the present invention provides a nonwoven fabric containing fibers produced by spinning a meltage prepared by melting and kneading a thermoplastic resin and a triglyceride. The production method of the nonwoven fabric of the present invention is not limited by the above method, and a triglyceride may be incorporated in fibers by another method.

The fibers included in the nonwoven fabric preferably have a relaxation time of 10 $\mu$s (microseconds) or more as determined by pulsed NMR of the fibers. Fibers having such a relaxation time are likely to cause bleed-out of the triglyceride from the inside, and thus a nonwoven fabric containing the fibers has strong barrier properties to liquid. A typical approach for enhancing the bleed-out properties is modifying crystallizability; however, a phenomenon has been observed in which fibers having substantially the same crystallinity may have different bleed-out properties. Intensive studies by the inventors of the present invention have revealed that, in addition to the crystallinity, relaxation time is an important factor of the bleed-out properties. In view of more markedly enhancing the advantage, the relaxation time is more preferably 12 $\mu$s or more, even more preferably 15 $\mu$s or more, and further preferably 20 $\mu$s or more. The relaxation time is preferably longer in view of accelerating the bleed-out of the triglyceride. Hence, the relaxation time has no upper limit, but the intended effect of the present invention is sufficiently exhibited by a relaxation time as long as about 80 $\mu$s.

In the present invention, the barrier properties of the nonwoven fabric to liquid can be improved even when the amount of the triglyceride contained in fibers is small, and bleed-out of a triglyceride is therefore unlikely to cause disadvantages.

To adjust the relaxation time in pulsed NMR within the above range, a random propylene copolymer and a low stereoregularity homopolypropylene may be blended, for example.

The crystallinity and the relaxation time in pulsed NMR are determined by the methods described later in examples.

The thickness of the fibers included in the nonwoven fabric of the present invention is preferably 40 μm or less, more preferably 30 μm or less, and even more preferably 20 μm or less in view of improving the barrier properties of the nonwoven fabric to liquid.

A nonwoven fabric including thinner fibers has stronger barrier properties to liquid, but such a nonwoven fabric may be difficult to produce, or such a nonwoven fabric may be produced inefficiently. The thickness is thus preferably 0.1 μm or more, more preferably 0.3 μm or more, and even more preferably 0.5 μm or more, in terms of the practical lower limit.

The thickness of fibers is determined in the following manner: a small piece is sampled from a nonwoven fabric; the fiber diameters of 70 fibers randomly selected are directly measured under a scanning electron microscope; and the average is calculated (2 significant digits).

The basis weight of the nonwoven fabric of the present invention is preferably 0.2 $g/m^2$ or more, more preferably 0.6 $g/m^2$ or more, and even more preferably 1.0 $g/m^2$ or more in view of improving the barrier properties of the nonwoven fabric to liquid.

A nonwoven fabric having a larger basis weight has stronger barrier properties to liquid but may have poor texture or low economic efficiency. The basis weight is thus preferably 50 $g/m^2$ or less, more preferably 35 $g/m^2$ or less, and even more preferably 20 $g/m^2$ or less, in terms of the practical upper limit.

The basis weight of a nonwoven fabric is a value obtained by dividing the weight of the nonwoven fabric by the area of the nonwoven fabric. The basis weight is preferably calculated by using a sample at least having an area of 1 $cm^2$ or more. Measurement is more preferably made on three or more pieces for a single sample, taking the texture of the nonwoven fabric into account, to calculate the average as the basis weight of the sample.

The nonwoven fabric of the present invention may consist of the fibers containing the polyolefin resin and the triglyceride or may include such fibers and other fibers. In the latter case, the nonwoven fabric of the present invention may include a blend fabric of the above-described fibers and the other fibers or may include a laminate of a layer consisting of the above-described fibers and a layer consisting of the other fibers. For example, the nonwoven fabric may be a laminate of a meltblown layer consisting of the above-described fibers and spunbond layers provided on the respective faces of the meltblown layer and containing the other fibers.

In particular, the nonwoven fabric of the present invention preferably has a meltblown layer and a spunbond layer, and the meltblown layer preferably includes fibers containing: a random propylene copolymer or a low stereoregularity homopolypropylene; a homopolypropylene; and the above-described triglyceride, in view of improving the barrier properties to liquid, especially in view of effectively suppressing seepage of liquid under pressure. The spunbond layer less contributes to the barrier properties. Hence, the constituent fibers thereof do not necessarily need to contain the above-described triglyceride, and the spunbond layer may consist of fibers not containing the triglyceride.

When the nonwoven fabric of the present invention includes the above-described fibers and the other fibers, the proportion of the above-described fibers in the nonwoven fabric is preferably 3% by mass or more, more preferably 5% by mass or more, and even more preferably 10% by mass or more, and is preferably 50% by mass or less, more preferably 45% by mass or less, and even more preferably 40% by mass or less, in view of improving the barrier properties of the nonwoven fabric to liquid regardless of the form of the nonwoven fabric, especially in view of successfully preventing the liquid seepage under pressure.

The nonwoven fabric of the present invention has strong barrier properties to liquid, especially to water, and thus is suitably used in applications for which waterproof properties are required. For example, the nonwoven fabric of the present invention is applicable to back sheets and leakproof sheets, such as leg cuffs and wings, for absorbent articles such as disposable diapers and sanitary napkins, waterproof sheets for rain gear, sticking plasters, wound dressings, medical isolation gowns, surgical gowns, surgical drapes and covers, and surgery caps.

When the nonwoven fabric of the present invention is used as a leakproof sheet for an absorbent article, the nonwoven fabric may be directly used as the leakproof sheet, or the nonwoven fabric may be used in combination with another sheet material.

In an embodiment, the nonwoven fabric of the present invention may be used as a filtration medium through which various fluids are to pass or as a part of the filtration medium. The nonwoven fabric of the present invention may be used as a filter for a mask or the like.

The above description is for a nonwoven fabric including fibers containing a particular triglyceride. According to the present invention, a water repellent composition that contains a particular triglyceride and is to be contained in a resin is also provided. The water repellent composition is kneaded into a melted resin and is used to impart water repellency to a shaped body formed of the resin. The water repellent composition contains a triglyceride, and the triglyceride includes

(a) a mixture of a triglyceride at least containing, in one molecule, a group derived from a fatty acid having 16 carbon atoms and a triglyceride at least containing, in one molecule, a group derived from a fatty acid having 18 carbon atoms, or

(b) a triglyceride at least containing, in one molecule, a group derived from a fatty acid having 16 carbon atoms and a group derived from a fatty acid having 18 carbon atoms.

In an embodiment, the water repellent composition of the present invention is to be contained in fibers and is used to impart water repellency to the fibers.

Specifically, in a shaped body such as fibers and films formed of a resin that contains the water repellent composition of the present invention, even when the water repellent composition on the surface of the shaped body falls by abrasion or the like, bleed-out of the water repellent composition from the inside of the shaped body to the surface recovers an intended water repellency, and thus the water repellency on the surface of the shaped body can be maintained for a long time. In particular, when the resin of a shaped body is a polyolefin resin containing a random propylene copolymer or a low stereoregularity homopolypropylene and a homopolypropylene, especially when the resin is a polyolefin resin containing a random propylene copolymer and a homopolypropylene, bleed-out of the water repellent composition is more certainly caused, and the shaped body more certainly exhibits the water repellency.

In another embodiment, the water repellent composition of the present invention is blended into a resin for rain gear, a sticking plaster, a wound dressing, a medical isolation gown, a surgery gown, a surgery drape and cover, an operating gown, a surgery cap, artificial leather, synthetic leather, suede, an umbrella, a tent, an outdoor garment, or the like and is used to impart water repellency to such a product.

In particular, the water repellent composition of the present invention is preferably for fibers made of a thermoplastic resin having capability to be formed into fibers, and especially, the water repellent composition is preferably for fibers made of a thermoplastic resin containing a polyolefin resin. In particular, the polyolefin resin preferably contains: a random propylene copolymer or a low stereoregularity homopolypropylene; and a homopolypropylene, and especially preferably contains a random propylene copolymer and a homopolypropylene, since bleed-out of the water repellent composition is more certainly caused as described above.

Details of the triglyceride contained in the water repellent composition of the present invention are the same as those of the above-described triglyceride contained in the nonwoven fabric of the present invention, and the description for the triglyceride contained in the nonwoven fabric is appropriately applied.

## Examples

The present invention will next be described in further detail by way of examples. However, the scope of the invention is not limited to the examples. Unless otherwise specified, "%" means "% by mass".

### [Example 1-1]

In the example, the water repellency of a water repellent composition containing a triglyceride was evaluated on a film containing the triglyceride by the following method. The result is shown in Table 1.

(1) Production of resin

A propylene homopolymer (HP461Y, manufactured by Lyondellbasel, hereinafter called "homoPP"), a random propylene copolymer (Vistamaxx 8880, manufactured by Exxonmobil, hereinafter called "randomPP"), and a triglyceride were weighed at a mass ratio of 63/27/10 and were placed in Labo Plastomill (manufactured by Toyo Seiki Seisaku-sho). The whole was kneaded at 180°C at 30 rpm for 10 minutes. After kneading, the resulting lump of resin was collected. The randomPP had an ethylene content of 8%.

The used triglyceride was a mixture of glyceryl tripalmitate and glyceryl tristearate. The mixing ratio was as shown in Table 1.

(2) Production of film

Using a LABO PRESS (manufactured by Toyo Seiki Seisaku-sho), the lump of resin was pressed at 180°C at 100 kgf for 1 minute and then cold pressed at ordinary temperature at 100 kgf for 1 minute, to obtain a film. A 0.5-mm spacer was used to adjust the film thickness to 0.5 mm.

(3) Evaluation of water repellency

The prepared film was immersed in 50 mL of a toluene solution, and the film surface was then wiped with tissue paper and was washed. The washed film was placed in an electric dryer and stored at 40°C for 7 days. After storage, the contact angle of the film surface was determined using a contact angle meter (Drop Master 500, manufactured by Kyowa

Interface Science).

- · Measurement method: drop method
- · Solution for Evaluation: test solution for wet tension (surface tension at 25°C: 35.0 mN/m; manufactured by Kanto Chemical)
- · Volume dropped: 2 μL
- · The contact angle 10 seconds after arrival of the drop was measured at five positions for a single sample, and the average of the contact angles at the five drops was used as the contact angle.

[Example 1-2]

A film was prepared in the same manner as in Example 1-1 and the water repellency thereof was evaluated, except that glyceryl tripalmitate and glyceryl tristearate were used at the mixing ratio shown in Table 1.

[Example 1-3]

A film was prepared in the same manner as in Example 1-1 and the water repellency thereof was evaluated, except that fully hydrogenated palm oil (manufactured by Ueda Oils & Fats) was used as the triglyceride. This triglyceride was a mixture of a plurality of triglycerides, and the distribution of the number of carbon atoms of groups derived from fatty acids in the triglyceride was as shown in Table 1.

[Example 1-4]

A film was prepared in the same manner as in Example 1-1 and the water repellency thereof was evaluated, except that glyceryl tristearate (technical grade, manufactured by Sigma-Aldrich) was used as the triglyceride. The proportion of the group derived from a fatty acid having 16 carbon atoms and the proportion of the group derived from a fatty acid having 18 carbon atoms in the triglyceride were as shown in Table 1.

[Example 1-5]

A film was prepared in the same manner as in Example 1-1 and the water repellency thereof was evaluated, except that hydrogenated high erucic rapeseed oil (manufactured by Yokozeki-yushi) was used as the triglyceride. The triglyceride was a mixture of a plurality of triglycerides, and the distribution of the number of carbon atoms of groups derived from fatty acids in the triglyceride was as shown in Table 1.

[Comparative Example 1-1]

A film was prepared in the same manner as in Example 1-1 and the water repellency thereof was evaluated, except that glyceryl tripalmitate was used as the triglyceride.

[Comparative Example 1-2]

A film was prepared in the same manner as in Example 1-1 and the water repellency thereof was evaluated, except that glyceryl tristearate was used as the triglyceride.

[Table 1]

| | Example | | | | | Comparative Example | |
|---|---|---|---|---|---|---|---|
| | 1-1 | 1-2 | 1-3 | 1-4 | 1-5 | 1-1 | 1-2 |
| HomoPP | 63 | 63 | 63 | 63 | 63 | 63 | 63 |
| RandomPP | 27 | 27 | 27 | 27 | 27 | 27 | 27 |
| Triglyceride | 10 | 10 | 10 | 10 | 10 | 10 | 10 |

(continued)

| | | Example | | | | | Comparative Example | |
|---|---|---|---|---|---|---|---|---|
| | | 1-1 | 1-2 | 1-3 | 1-4 | 1-5 | 1-1 | 1-2 |
| Proportion of group derived from fatty acid [% by mass] | C12 | - | - | - | - | - | - | - |
| | C14 | - | - | 1 | - | - | - | - |
| | C16 | 40 | 30 | 42 | 29 | 3 | 100 | - |
| | C18 | 60 | 70 | 57 | 62 | 37 | - | 100 |
| | C20 | - | - | - | - | 9 | - | - |
| | C22 | - | - | - | - | 50 | - | - |
| | C24 | - | - | - | - | 1 | - | - |
| | Unsaturated | None | None | None | None | None | None | None |
| Contact angle [°] | | 97 | 98 | 102 | 108 | 90 | 78 | 83 |

The results shown in Table 1 reveal that the films in Examples have higher contact angles with the solution for evaluation, in contrast to the films in Comparative Examples.

[Example 2-1]

(1) Production of masterbatch

HomoPP and a triglyceride were weighed at a mass ratio of 90/10 and were placed in a twin-screw extruder (manufactured by Toyo Seiki Seisaku-sho). The whole was kneaded to give a masterbatch.
The triglyceride used was the same as used in Example 1-3.

(2) Production of meltblown nonwoven fabric

The resulting masterbatch was used as the material to prepare a meltblown nonwoven fabric.
The basis weight of the meltblown nonwoven fabric and the fiber diameter of the constituent fibers were as shown in Table 2.
The basis weight of the meltblown nonwoven fabric was determined in the following manner: 10 test pieces (16 cm × 16 cm) were randomly sampled from the nonwoven fabric, and were weighed; and the average mass of the test pieces was divided by 0.0256.
On the respective faces of the meltblown nonwoven fabric prepared as above, spunbond nonwoven fabrics shown in Table 2 were laminated to obtain an SMS nonwoven fabric.

[Examples 2-2 to 2-12]

HomoPP and randomPP were used as the polyolefin resin. The proportions of the homoPP, the randomPP, and the triglyceride were as shown in Table 2 and Table 3.
In Example 2-6, the same triglyceride as in Example 1-4 was used as the triglyceride.
In Example 2-7, the same triglyceride as in Example 1-5 was used as the triglyceride.
In Example 2-8, fully hydrogenated soybean oil was used as the triglyceride.
An SMS nonwoven fabric was prepared in the same manner as in Example 2-1 except for the above.

[Comparative Example 2-1]

HomoPP and randomPP were used as the polyolefin resin. No triglyceride was used. The proportions of the homoPP and the randomPP were as shown in Table 3.
An SMS nonwoven fabric was prepared in the same manner as in Example 2-1 except for the above.

[Evaluation 1]

The relaxation times of the meltblown layers in the SMS nonwoven fabrics produced in Examples 2-1, 2-4, 2-6,

and 2-7 and Comparative Example 2-1 were determined by pulsed NMR in the following manner. The results are shown in Table 2.

- · Apparatus used: the minispec mq20, manufactured by Bruker
- · Measurement method: Solid-echo method
- · 90° Pulse width: 2.1 microseconds
- · Repetition time: 1 second
- · Number of scans: 32
- · Measurement temperature: 30°C
- · Analysis method: non-linear least-squares method

A sample was packed in a sample tube, and the $T_2$ relaxation time of $^1H$ was determined. For one peak of the relaxation time obtained by analyzing the sample, components different in relaxation time were subjected to peak deconvolution and two components, specifically, a component having a short relaxation time (S) and a component having a long relaxation time (L) were separately detected. The proton ratios H(S) [%] and H(L) [%] and the relaxation times $T_2(S)$ and $T_2(L)$ of the respective components were determined. The measurement was performed in quadruplicate, and the averages were used as the measurement results. The found proton ratios and relaxation times of the two components were used to calculate the relaxation time of each sample using the following equation.

$$\text{Relaxation time [microseconds]} = \{<H(S)/100> \times T_2(S)\} + \{<H(L)/100> \times T_2(L)\}$$

[Evaluation 2]

The degrees of liquid seepage of the SMS nonwoven fabrics prepared in Examples and Comparative Examples were evaluated in the following manner. The results are shown in Table 2 and Table 3.

On a filter paper (No. 2, a diameter of 70 mm, manufactured by ADVANTEC), a sample (8 cm × 8 cm) cut from an SMS nonwoven fabric was placed. On the nonwoven fabric, a cut piece (8 cm × 8 cm) of a dry pulp sheet (Reed Healthy-Cooking Paper Double (trade name), manufactured by Lion, a basis weight of 40 g/m$^2$) was placed.

At the central part of the dry pulp sheet, 1 g of a test solution for wet tension (surface tension at 25°C: 35.0 mN/m) was injected with a dropper. After injection, an acrylic plate having a diameter of 60 mm was stacked, and a weight was placed thereon to press the sheets at 7 kPa for 1 hour.

After 1 hour, the weight was removed, and the area of the solution seeping into the filter paper was determined using an image analyzer. The measurement was performed in duplicate, and the total of the areas of the solution seeping into the filter paper was used as the seepage area of the sample.

[Table 2]

| | | Example | | | | | | | |
|---|---|---|---|---|---|---|---|---|---|
| | | 2-1 | 2-2 | 2-3 | 2-4 | 2-5 | 2-6 | 2-7 | 2-8 |
| Spunbond | HomoPP (%) | 100 | 100 | 100 | 100 | 100 | 100 | 100 | 100 |
| | Basis weight [g/m$^2$] | 9 | 9 | 9 | 9 | 9 | 9 | 9 | 9 |
| Meltblown | HomoPP (%) | 90.0 | 81.0 | 72.0 | 63.0 | 54.0 | 63.0 | 63.0 | 63.0 |
| | RandomPP (%) | - | 9.0 | 18.0 | 27.0 | 36.0 | 27.0 | 27.0 | 27.0 |
| | Triglyceride (%) | 10.0 | 10.0 | 10.0 | 10.0 | 10.0 | 10.0 | 10.0 | 10.0 |
| | Basis weight [g/m$^2$] | 10 | 10 | 10 | 10 | 10 | 10 | 10 | 10 |
| Spunbond | HomoPP (%) | 100 | 100 | 100 | 100 | 100 | 100 | 100 | 100 |
| | Basis weight [g/m$^2$] | 9 | 9 | 9 | 9 | 9 | 9 | 9 | 9 |

(continued)

| | | Example | | | | | | | |
|---|---|---|---|---|---|---|---|---|---|
| | | 2-1 | 2-2 | 2-3 | 2-4 | 2-5 | 2-6 | 2-7 | 2-8 |
| Proportion of group derived from fatty acid [% by mass] | C12 | - | - | - | - | - | - | - | - |
| | C14 | 1 | 1 | 1 | 1 | 1 | - | - | - |
| | C16 | 42 | 42 | 42 | 42 | 42 | 29 | 3 | 11 |
| | C18 | 57 | 57 | 57 | 57 | 57 | 62 | 37 | 88 |
| | C20 | - | - | - | - | - | - | 9 | 1 |
| | C22 | - | - | - | - | - | - | 50 | - |
| | C24 | - | - | - | - | - | - | 1 | - |
| | Unsaturated | None | None | None | None | None | None | None | None |
| Fiber diameter [μm] | | 1.5 | 1.5 | 1.4 | 1.1 | 1.6 | 1.4 | 1.7 | 1.4 |
| Relaxation time [μs] | | 12 | | | 22 | | 22 | 22 | |
| Seepage area [$mm^2$] | | 3846.5 | 10.5 | 0.7 | 0 | 142.5 | 0 | 1.4 | 12.5 |

[Table 3]

| | | Example | | | | Comparative Example |
|---|---|---|---|---|---|---|
| | | 2-9 | 2-10 | 2-11 | 2-12 | 2-1 |
| Spunbond | HomoPP (%) | 100 | 100 | 100 | 100 | 100 |
| | Basis weight [$g/m^2$] | 9 | 9 | 9 | 9 | 9 |
| Meltblown | HomoPP (%) | 64.4 | 63.7 | 60.2 | 57.4 | 70.0 |
| | RandomPP (%) | 27.6 | 27.3 | 25.8 | 24.6 | 30.0 |
| | Triglyceride (%) | 8.0 | 9.0 | 14.0 | 18.0 | 0 |
| | Basis weight [$g/m^2$] | 10 | 10 | 10 | 10 | 10 |
| Spunbond | HomoPP (%) | 100 | 100 | 100 | 100 | 100 |
| | Basis weight [$g/m^2$] | 9 | 9 | 9 | 9 | 9 |
| Proportion of group derived from fatty acid [% by mass] | C12 | - | - | - | - | - |
| | C14 | 1 | 1 | 1 | 1 | - |
| | C16 | 42 | 42 | 42 | 42 | - |
| | C18 | 57 | 57 | 57 | 57 | - |
| | C20 | - | - | - | - | - |
| | C22 | - | - | - | - | - |
| | C24 | - | - | - | - | - |
| | Unsaturated | None | None | None | None | None |
| Fiber diameter [μm] | | 1.4 | 1.3 | 1.5 | 1.7 | 1.1 |
| Relaxation time [μs] | | | | | | 12 |
| Seepage area [$mm^2$] | | 3846.5 | 0 | 0 | 2.2 | 7693 |

The results shown in Table 2 and Table 3 clearly reveal that each SMS nonwoven fabric produced in Examples, which contains a small amount of triglyceride, has strong barrier properties to a liquid having a low surface tension.

In particular, comparison between Example 2-1 and Examples 2-2 to 2-5 clearly reveals that using a combination of homoPP and randomPP as the polyolefin resin included in the meltblown nonwoven fabric improves the barrier properties to a liquid having a low surface tension as compared with using homoPP alone. In particular, it is revealed that by

incorporating randomPP in an amount of 15% by mass or more and 30% by mass or less based on the fibers included in the meltblown nonwoven fabric, the barrier properties to a liquid having a low surface tension are extremely improved.

In addition, the results of Example 2-9 to Example 2-12 clearly reveal that by incorporating triglyceride in an amount of 8% by mass or more and 18% by mass or less based on the fibers included in the meltblown nonwoven fabric, the barrier properties to a liquid having a low surface tension are improved. In particular, it is revealed that by incorporating triglyceride in an amount of 9% by mass or more and 14% by mass or less, the barrier properties to a liquid having a low surface tension are extremely improved.

The results of Examples 2-1 and 2-4 reveal that fibers having a longer relaxation time more improve the barrier properties of a nonwoven fabric than that having a shorter relaxation time, on condition that both contain the same type of triglyceride.

Comparison between Example 2-4 and Examples 2-6 to 2-8 reveals that by incorporating a triglyceride having 20% by mass or more and 50% by mass or less of a group derived from a fatty acid having 16 carbon atoms and 50% by mass or more and 70% by mass or less of a group derived from a fatty acid having 18 carbon atoms based on the total amount of groups derived from fatty acids contained in the triglyceride, the barrier properties to a liquid having a low surface tension are extremely improved.

Industrial Applicability

According to the present invention, a nonwoven fabric having stronger barrier properties to liquid than conventional nonwoven fabrics is provided, as described above in detail.

## Claims

**1.** A nonwoven fabric comprising fibers containing: a thermoplastic resin having capability to be formed into fibers; and a triglyceride, wherein

the thermoplastic resin includes a polyolefin resin,
the triglyceride includes

(a) a mixture of a triglyceride at least containing, in one molecule, a group derived from a fatty acid having 16 carbon atoms and a triglyceride at least containing, in one molecule, a group derived from a fatty acid having 18 carbon atoms, or
(b) a triglyceride at least containing, in one molecule, a group derived from a fatty acid having 16 carbon atoms and a group derived from a fatty acid having 18 carbon atoms.

**2.** The nonwoven fabric according to claim 1, wherein the triglyceride contains no group derived from an unsaturated fatty acid.

**3.** The nonwoven fabric according to claim 1 or 2, wherein the triglyceride at least contains, in one molecule, a group derived from a fatty acid having 16 carbon atoms and a group derived from a fatty acid having 18 carbon atoms.

**4.** The nonwoven fabric according to any one of claims 1 to 3, wherein a proportion of the group derived from a fatty acid having 16 carbon atoms is not less than 1% by mass and less than 55% by mass, and a proportion of the group derived from a fatty acid having 18 carbon atoms is not less than 5% by mass and less than 90% by mass, in a total amount of groups derived from fatty acids contained in all the triglycerides contained in the fibers.

**5.** The nonwoven fabric according to any one of claims 1 to 4, wherein a proportion of the group derived from a fatty acid having 16 carbon atoms is 25% by mass or more and 45% by mass or less, and a proportion of the group derived from a fatty acid having 18 carbon atoms is 55% by mass or more and 65% by mass or less, in the total amount of groups derived from fatty acids contained in all the triglycerides contained in the fibers.

**6.** The nonwoven fabric according to any one of claims 1 to 5, wherein a mass ratio of the group derived from a fatty acid having 18 carbon atoms to the group derived from a fatty acid having 16 carbon atoms in all the triglycerides contained in the fibers is 1.0 or more and 15.0 or less.

**7.** The nonwoven fabric according to any one of claims 1 to 6, wherein the fibers have a relaxation time of 10 $\mu$s or more as determined by pulsed NMR.

**8.** The nonwoven fabric according to any one of claims 1 to 7, wherein the nonwoven fabric comprises a meltblown nonwoven fabric or a composite nonwoven fabric having a meltblown layer.

**9.** The nonwoven fabric according to any one of claims 1 to 8, wherein the nonwoven fabric at least contains a spunbond layer and a meltblown layer,

a mass ratio of the spunbond layer to a whole of the nonwoven fabric is 50% by mass or more and 95% by mass or less, and
a mass ratio of the meltblown layer to the whole of the nonwoven fabric is 5% by mass or more and 50% by mass or less.

**10.** The nonwoven fabric according to any one of claims 1 to 9, wherein the polyolefin resin is a polypropylene.

**11.** The nonwoven fabric according to any one of claims 1 to 10, wherein the triglyceride is contained in an amount of 1% by mass or more and 30% by mass or less based on the fibers containing the triglyceride.

**12.** The nonwoven fabric according to any one of claims 1 to 11, wherein a proportion of the fibers containing the triglyceride is 3% by mass or more in the nonwoven fabric.

**13.** The nonwoven fabric according to any one of claims 1 to 12, wherein the polyolefin resin contains: a random propylene copolymer or a low stereoregularity homopolypropylene; and a homopolypropylene.

**14.** The nonwoven fabric according to claim 13, wherein a proportion of the random propylene copolymer or the low stereoregularity homopolypropylene in the fibers is not less than 5% by mass and less than 60% by mass.

**15.** The nonwoven fabric according to any one of claims 1 to 14, wherein the nonwoven fabric has a meltblown layer and a spunbond layer, and
the meltblown layer includes fibers containing: a random propylene copolymer or a low stereoregularity homopolypropylene; a homopolypropylene; and the triglyceride.

**Patentansprüche**

**1.** Ein Vliesstoff, umfassend Fasern, enthaltend: ein thermoplastisches Harz das dazu geeignet ist zu Fasern geformt zu werden; und ein Triglycerid, wobei

das thermoplastische Harz ein Polyolefinharz beinhaltet,
das Triglycerid

(a) ein Gemisch von einem Triglycerid, das, in einem Molekül, mindestens eine Gruppe enthält, die von einer Fettsäure mit 16 Kohlenstoffatomen abgeleitet ist, und einem Triglycerid, das, in einem Molekül, mindestens eine Gruppe enthält, die von einer Fettsäure mit 18 Kohlenstoffatomen abgeleitet ist oder
(b) ein Triglycerid, das, in einem Molekül, mindestens eine Gruppe enthält, die von einer Fettsäure mit 16 Kohlenstoffatomen abgeleitet ist, und eine Gruppe, die von einer Fettsäure mit 18 Kohlenstoffatomen abgeleitet ist,

beinhaltet.

**2.** Der Vliesstoff gemäß Anspruch 1, wobei das Triglycerid keine Gruppe enthält, die von einer ungesättigten Fettsäure abgeleitet ist.

**3.** Der Vliesstoff gemäß Anspruch 1 oder 2, wobei das Triglycerid, in einem Molekül, mindestens eine Gruppe enthält, die von einer Fettsäure mit 16 Kohlenstoffatomen abgeleitet ist, und eine Gruppe, die von einer Fettsäure mit 18 Kohlenstoffatomen abgeleitet ist.

**4.** Der Vliesstoff gemäß einem der Ansprüche 1 bis 3, wobei ein Anteil der Gruppe, die von einer Fettsäure mit 16 Kohlenstoffatomen abgeleitet ist, nicht weniger als 1 Massen-% und weniger als 55 Massen-% beträgt, und ein Anteil der Gruppe, die von einer Fettsäure mit 18 Kohlenstoffatomen abgeleitet ist, nicht weniger als 5 Massen-% und

weniger als 90 Massen-% beträgt, in einer Gesamtmenge der Gruppen, die von Fettsäuren abgeleitet sind, die in allen Triglyceriden, die in den Fasern enthalten sind, enthalten sind.

**5.** Der Vliesstoff gemäß einem der Ansprüche 1 bis 4, wobei ein Anteil der Gruppe, die von einer Fettsäure mit 16 Kohlenstoffatomen abgeleitet ist, 25 Massen-% oder mehr und 45 Massen-% oder weniger beträgt, und ein Anteil der Gruppe, die von einer Fettsäure mit 18 Kohlenstoffatomen abgeleitet ist, 55 Massen-% oder mehr und 65 Massen-% oder weniger beträgt, bezogen auf eine Gesamtmenge der Gruppen, die von Fettsäuren abgeleitet sind, die in allen Triglyceriden, die in den Fasern enthaltenen sind, enthalten sind.

**6.** Der Vliesstoff gemäß einem der Ansprüche 1 bis 5, wobei ein Massenverhältnis der Gruppe, die von einer Fettsäure mit 18 Kohlenstoffatomen abgeleitet ist, zu der Gruppe, die von einer Fettsäure mit 16 Kohlenstoffatomen abgeleitet ist, in allen Triglyceriden, die in den Fasern enthalten sind, 1,0 oder mehr und 15,0 oder weniger beträgt.

**7.** Der Vliesstoff gemäß einem der Ansprüche 1 bis 6, wobei die Fasern eine Relaxationszeit von 10 $\mu$s oder mehr aufweisen, wie durch gepulste NMR bestimmt.

**8.** Der Vliesstoff gemäß einem der Ansprüche 1 bis 7, wobei der Vliesstoff einen schmelzgeblasenen Vliesstoff oder einen Verbundvliesstoff mit einer schmelzgeblasenen Schicht umfasst.

**9.** Der Vliesstoff gemäß einem der Ansprüche 1 bis 8, wobei der Vliesstoff mindestens eine spinngebundene Schicht und eine schmelzgeblasene Schicht enthält,

ein Massenverhältnis der spinngebundenen Schicht zum gesamten Vliesstoff 50 Massen-% oder mehr und 95 Massen-% oder weniger beträgt, und
ein Massenverhältnis der schmelzgeblasenen Schicht zum gesamten Vliesstoff 5 Massen-% oder mehr und 50 Massen-% oder weniger beträgt.

**10.** Der Vliesstoff gemäß einem der Ansprüche 1 bis 9, wobei das Polyolefinharz ein Polypropylen ist.

**11.** Der Vliesstoff gemäß einem der Ansprüche 1 bis 10, wobei das Triglycerid in einer Menge von 1 Massen-% oder mehr und 30 Massen-% oder weniger, bezogen auf die Fasern, die das Triglycerid enthalten, enthalten ist.

**12.** Der Vliesstoff gemäß einem der Ansprüche 1 bis 11, wobei ein Anteil der Fasern, die das Triglycerid enthalten, 3 Massen-% oder mehr im Vliesstoff beträgt.

**13.** Der Vliesstoff gemäß einem der Ansprüche 1 bis 12, wobei das Polyolefinharz enthält: ein statistisches Propylen-Copolymer oder ein Homopolypropylen mit geringer Stereoregularität; und ein Homopolypropylen.

**14.** Der Vliesstoff gemäß Anspruch 13, wobei ein Anteil des statistischen Propylen-Copolymers oder des Homopolypropylens mit geringer Stereoregularität in den Fasern nicht weniger als 5 Massen-% und weniger als 60 Massen-% beträgt.

**15.** Der Vliesstoff gemäß einem der Ansprüche 1 bis 14, wobei der Vliesstoff eine schmelzgeblasene Schicht und eine spinngebundene Schicht aufweist und
die schmelzgeblasene Schicht Fasern beinhaltet, die enthalten: ein statistisches Propylen-Copolymer oder ein Homopolypropylen mit geringer Stereoregularität; ein Homopolypropylen; und das Triglycerid.

## Revendications

**1.** Etoffe non tissée comprenant des fibres contenant : une résine thermoplastique ayant la capacité d'être mise sous la forme de fibres ; et un triglycéride, dans laquelle

la résine thermoplastique inclut une résine de polyoléfine,
le triglycéride inclut

(a) un mélange d'un triglycéride au moins contenant, dans une molécule, un groupe dérivé d'un acide gras ayant 16 atomes de carbone et d'un triglycéride au moins contenant, dans une molécule, un groupe dérivé

d'un acide gras ayant 18 atomes de carbone, ou
(b) un triglycéride au moins contenant, dans une molécule, un groupe dérivé d'un acide gras ayant 16 atomes de carbone et un groupe dérivé d'un acide gras ayant 18 atomes de carbone.

**2.** Etoffe non tissée selon la revendication 1, dans laquelle le triglycéride ne contient pas de groupe dérivé d'un acide gras insaturé.

**3.** Etoffe non tissée selon la revendication 1 ou 2, dans laquelle le triglycéride au moins contient, dans une molécule, un groupe dérivé d'un acide gras ayant 16 atomes de carbone et un groupe dérivé d'un acide gras ayant 18 atomes de carbone.

**4.** Etoffe non tissée selon l'une quelconque des revendications 1 à 3, dans laquelle la proportion du groupe dérivé d'un acide gras ayant 16 atomes de carbone n'est pas inférieure à 1 % en masse et est inférieure à 55 % en masse, et la proportion du groupe dérivé d'un acide gras ayant 18 atomes de carbone n'est pas inférieure à 5 % en masse et est inférieure à 90 % en masse, de la quantité totale de groupes dérivés d'acides gras contenus dans tous les triglycérides contenus dans les fibres.

**5.** Etoffe non tissée selon l'une quelconque des revendications 1 à 4, dans laquelle la proportion du groupe dérivé d'un acide gras ayant 16 atomes de carbone est de 25 % en masse ou plus et 45 % en masse ou moins, et la proportion du groupe dérivé d'un acide gras ayant 18 atomes de carbone est de 55 % en masse ou plus et 65 % en masse ou moins, de la quantité totale de groupes dérivés d'acides gras contenus dans tous les triglycérides contenus dans les fibres.

**6.** Etoffe non tissée selon l'une quelconque des revendications 1 à 5, dans laquelle le rapport en masse du groupe dérivé d'un acide gras ayant 18 atomes de carbone au groupe dérivé d'un acide gras ayant 16 atomes de carbone dans tous les triglycérides contenus dans les fibres est de 1,0 ou plus et 15,0 ou moins.

**7.** Etoffe non tissée selon l'une quelconque des revendications 1 à 6, dans laquelle les fibres ont un temps de relaxation de 10 $\mu$s ou plus, tel que déterminé par RMN impulsionnelle.

**8.** Etoffe non tissée selon l'une quelconque des revendications 1 à 7, laquelle étoffe non tissée comprend une étoffe non tissée soufflée à l'état fondu ou une étoffe non tissée composite ayant une couche soufflée à l'état fondu.

**9.** Etoffe non tissée selon l'une quelconque des revendications 1 à 8, laquelle étoffe non tissée au moins contient une couche filée-liée et une couche soufflée à l'état fondu,

le rapport en masse de la couche filée-liée à la totalité de l'étoffe non tissée est de 50 % en masse ou plus et 95 % en masse ou moins, et
le rapport en masse de la couche soufflée à l'état fondu à la totalité de l'étoffe non tissée est de 5 % en masse ou plus et 50 % en masse ou moins.

**10.** Etoffe non tissée selon l'une quelconque des revendications 1 à 9, dans laquelle la résine de polyoléfine est un polypropylène.

**11.** Etoffe non tissée selon l'une quelconque des revendications 1 à 10, dans laquelle le triglycéride est contenu en une quantité de 1 % en masse ou plus et 30 % en masse ou moins sur la base des fibres contenant le triglycéride.

**12.** Etoffe non tissée selon l'une quelconque des revendications 1 à 11, dans laquelle la proportion des fibres contenant le triglycéride est de 3 % en masse ou plus dans l'étoffe non tissée.

**13.** Etoffe non tissée selon l'une quelconque des revendications 1 à 12, dans laquelle la résine de polyoléfine contient : un copolymère de propylène aléatoire ou un homopolypropylène de faible stéréorégularité ; et un homopolypropylène.

**14.** Etoffe non tissée selon la revendication 13, dans laquelle la proportion du copolymère de propylène aléatoire ou de l'homopolypropylène de faible stéréorégularité dans les fibres n'est pas inférieure à 5 % en masse et est inférieure à 60 % en masse.

**15.** Etoffe non tissée selon l'une quelconque des revendications 1 à 14, laquelle étoffe non tissée a une couche soufflée à l'état fondu et une couche filée-liée,

et la couche soufflée à l'état fondu inclut des fibres contenant : un copolymère de propylène aléatoire ou un homopolypropylène de faible stéréorégularité ; un homopolypropylène ; et le triglycéride.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2012100772 A1
- US 2014272223 A1
- US 2020129345 A1
- US 2019070045 A1
- EP 2266514 A
- US 2012109090 A
- EP 2901977 A
- JP 2009227931 A
- JP 2004277467 A
- JP 2012530855 A
- JP 2013159884 A
- JP 2012531530 A
- JP 2019509406 A
- JP 2018503751 A
- JP S478932 A
- JP 2016089009 A